# EUROPEAN PATENT APPLICATION

(11) **EP 4 618 231 A1**
(43) Date of publication of application: **17.09.2025**
(21) Application number: 24898023.7
(22) Date of filing: 22.11.2024
(51) Int. Cl.: H01M 10/42, H05B 3/00, H01M 10/48, H01M 50/107

(54) **THERMAL PROPAGATION TEST METHOD FOR BATTERY CELL AND HEATER FOR IGNITING BATTERY CELL FOR THERMAL PROPAGATION TEST**

(30) Priority: 27.11.2023 KR 20230166466
(71) Applicant: LG Energy Solution, Ltd., Seoul 07335 (KR)
(72) Inventor: JEON, Byeongjun, Daejeon 34122 (KR); KEUM, Jong Yoon, Daejeon 34122 (KR); AN, Ji Myong, Daejeon 34122 (KR)
(74) Representative: Plasseraud IP
(86) International application number: PCT/KR2024/018586
(87) International publication number: WO 2025/116427

(57) **Abstract**

A heater for igniting a battery cell for testing thermal propagation may include a heating portion including a heater wire and a connecting portion connected to the heater wire. The heating portion may be disposed on a bottom surface of the batter cell, and the heating portion may be configured to heat the bottom surface of the battery cell. A method for testing thermal propagation using the heater may prevent a side rupture of the battery cell in a thermal propagation test, thereby preventing errors in the thermal propagation test.

## Description

### TECHNICAL FIELD

The present disclosure relates to a method for testing thermal propagation of a battery cell and a heater for igniting a battery cell (cylindrical trigger cell) for a thermal propagation test.

### BACKGROUND

Unlike primary batteries that cannot be recharged, secondary batteries may be charged and discharged and are applied not only to portable devices but also to electric vehicles (EVs) and hybrid electric vehicles (HEVs) driven by electrical power sources.

The types of secondary batteries that are currently widely used include lithium ion batteries, lithium polymer batteries, nickel cadmium batteries, nickel hydrogen batteries, nickel zinc batteries, etc. An operating voltage of a unit secondary battery cell, i.e., a unit battery cell, is approximately 2.5 V to 4.6 V. Therefore, when a higher output voltage is required, a plurality of battery cells are connected in series to configure a battery pack. In addition, a battery pack is configured by connecting a plurality of battery cells in parallel depending on the charge/discharge capacity required for a battery pack. Therefore, the number of battery cells included in the battery pack may be set to vary depending on the required output voltage or charge/discharge capacity.

When a battery pack is configured by connecting a plurality of battery cells in series/parallel, it is common to first configure a battery module including at least one battery cell, or a plurality of battery cells, and then configure a battery pack by using at least one such battery module and adding other components. Here, the battery module refers to a component in which a plurality of battery cells are connected in series or parallel, and the battery pack refers to a component in which a plurality of battery modules are connected in series or parallel to increase capacity and output, etc.

However, if such battery modules and packs are overcharged, etc., an explosion or fire may occur due to swelling of the battery module, and such explosion or fire may cause greater risks, which may even lead to casualties.

In addition, as the battery market grows, safety enhancement is required. There is a trend in international certification to require a thermal propagation (TP) test. In the TP test of a cylindrical cell, a method of igniting the cylindrical cell by heating the side of the cylindrical cell has been used in the related art. However, the related art method of igniting a cylindrical cell causes a side rupture of the cylindrical cell when the cylindrical cell is ignited.

According to the international certification ISO6469-1 standard, normal ignition of a trigger cell is required during the TP test, and a top cap of the cell has to be opened to be recognized as normal ignition. The occurrence of a side rupture of the cell during cell ignition is not recognized as normal ignition. Therefore, a TP test method that may ignite a cylindrical cell without causing a side rupture and development of a new heater is required.

### SUMMARY

### TECHNICAL PROBLEMS

According to an aspect of the present disclosure, the present disclosure provides a thermal propagation test method that prevents a side rupture when a cylindrical cell for a thermal propagation test is ignited and a heater for igniting a cylindrical trigger cell for a thermal propagation test.

### TECHNICAL SOLUTION

According to an aspect of the present disclosure, a heater for igniting a battery cell for a thermal propagation test includes: a heating portion disposed on a bottom surface of the battery cell to heat the bottom surface of the battery cell; and a connecting portion connected to a heater wire in the heating portion.

The heating portion may be formed in a circular shape.

The heating portion may cover at least a portion of the bottom surface of the battery cell.

The heating portion may include: a heating member heating the bottom surface of the battery cell; and a heat-resistant sheet disposed on one surface of the heating member.

The heating member may be formed of heating metal.

The heating metal may be Inconel.

The heat-resistant sheet may be disposed on each of upper and lower surfaces of the heating member.

The heat-resistant sheet may be a mica sheet.

The heating portion may be attached to the battery cell through a polyimide (PI) tape.

The heater may further include a first temperature sensor measuring a temperature of the heater.

The heater may further include a second temperature sensor measuring a temperature of the battery cell.

The battery cell may be a cylindrical battery cell.

According to an aspect of the present disclosure, a method for testing thermal propagation of a battery cell includes: disposing a heater on a bottom surface of the battery cell to heat a bottom surface of the battery cell; and igniting the battery cell by heating the bottom surface of the battery cell.

The method for testing thermal propagation of a battery cell may further include: causing a top cap of the battery cell to be opened after igniting the battery cell.

### ADVANTAGEOUS EFFECTS

The thermal propagation test method and the heater for igniting a battery cell according to an aspect of the present disclosure may prevent a side rupture when igniting a battery cell for a thermal propagation test, thereby preventing errors in a thermal propagation test.

### BRIEF DESCRIPTION OF THE DRAWINGS

FIG. 1 is a diagram illustrating the inside of a cylindrical battery cell,
FIG. 2 is a block diagram illustrating a heater for igniting a battery cell (cylindrical trigger cell) for a thermal propagation test according to an aspect of the present disclosure,
FIG. 3 is a diagram illustrating a side of the heater illustrated in FIG. 2,
FIG. 4 is a diagram illustrating a shape of the heater illustrated in FIG. 2,
FIG. 5 is a diagram illustrating an example in which a heater is coupled to a battery cell (cylindrical trigger cell) according to an aspect of the present disclosure,
FIG. 6 is a photograph of a battery cell (cylindrical trigger cell) after a test,
FIG. 7 is a photograph illustrating upper and lower portions of the battery cell (cylindrical trigger cell) in FIG. 6, in which FIG. 7(a) is a photograph illustrating the upper portion of the battery cell and FIG. 7(b) is a photograph illustrating the lower portion of the battery cell, and
FIG. 8 is a graph illustrating the results of testing thermal propagation of the battery cell (cylindrical trigger cell).

### DETAILED DESCRIPTION

The advantages and features of the present disclosure and the methods for achieving them will become apparent by referring to the aspects described in detail below together with the accompanying drawings. However, the present disclosure is not limited to the aspects disclosed below but may be implemented in various different forms, and these aspects are provided only to make the disclosure of the present disclosure complete and to fully inform those skilled in the art of the scope of the disclosure, and the present disclosure is defined only by the scope of the claims. Accordingly, in some aspects, well-known process steps, well-known device structures, and well-known techniques are not specifically described in order to avoid ambiguity in the interpretation of the present disclosure. Like reference numerals refer to like elements throughout the specification.

In the drawings, the thickness of layers, films, panels, regions, etc., are exaggerated for clarity. The various figures are thus not to scale. Like reference numerals designate like elements throughout the specification. It will be understood that when an element, such as a layer, film, region, or substrate, is referred to as being "on" another element, it may be directly on the other element or intervening elements may also be present. In contrast, when an element is referred to as being "directly on" another element, there are no intervening elements present. In addition, it will be understood that when an element, such as a layer, film, region, or substrate is referred to as being "below" another element, it may be directly below the other element or intervening elements may also be present. In contrast, when an element is referred to as being "directly under" another element, there are no intervening elements present.

Before describing a method for testing thermal propagation of a battery cell and a heater for igniting a battery cell for a thermal propagation test according to an aspect of the present disclosure, a battery cell is first described.

Battery cells may be classified into prismatic, cylindrical, and pouch-shaped types depending on the shape of cases.

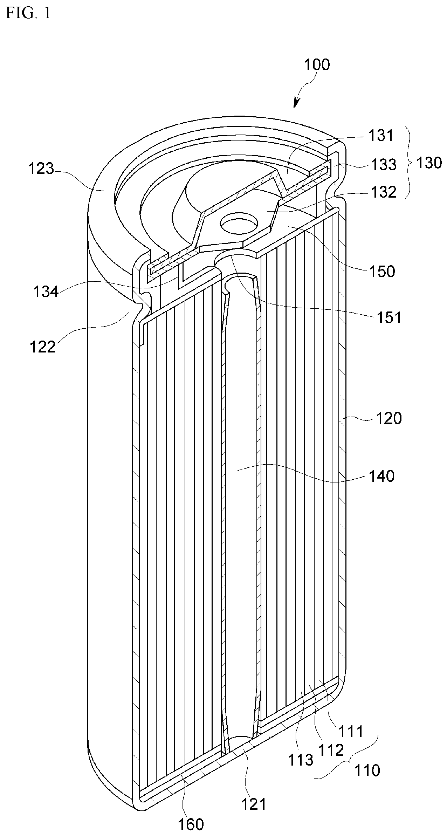

FIG. 1 is a diagram illustrating the inside of a cylindrical battery cell 100.

The cylindrical battery cell 100 may include a jelly roll-shaped electrode assembly 110 and a battery case 120 for storing the electrode assembly 110, an upper insulating member 150 may be disposed at an upper end of the electrode assembly 110, and a lower insulating member 160 may be disposed at a lower end of the electrode assembly 110.

The electrode assembly 110 may be a jelly roll-shaped structure in which a positive electrode 111, a negative electrode 113, and a separator 112 are interposed therebetween and wound, and a center pin 140 may be inserted into the center thereof.

The cylindrical battery cell 100 may be formed by housing the electrode assembly 110 in the battery case 120, injecting an electrolyte into the battery case 120, and then coupling a cap assembly 130 to the top of the battery case 120. The battery case 120 may be cylindrical, and the jelly roll-shaped electrode assembly 110 may be housed in the cylindrical battery case 120 to implement a cylindrical secondary battery.

The battery case 120 may include a bottom portion 121 disposed at the bottom, a beading portion 122, and a clamping portion 123.

The beading portion 122, for stable bonding of the cap assembly 130, may be formed in a circumferential direction in an upper surface of an outer circumferential surface of the battery case 120, and may be formed to be recessed in the center direction of the electrode assembly 110 on the outer circumferential surface of the battery case 120. The beading portion 122 may prevent movement of the electrode assembly 110.

The clamping portion 123 may be formed to be disposed at an upper portion of the beading portion 122 and wrap around the edge portion of the cap assembly 130 in the circumferential direction. The clamping portion 123 may promote stable bonding of the cap assembly 130.

The cap assembly 130 may include an upper cap 131 forming a positive electrode terminal, a cap plate 132 to which a positive electrode tab 134 extending from the electrode assembly 110 in an upward direction is connected, and a gasket 133 for maintaining airtightness.

The gasket 133 may be mounted on upper inner surfaces of the clamping portion 123 and the beading portion 122 to increase sealing force between the cap assembly 130 and the battery case 120.

As described above, the positive electrode tab 134 may extend from the electrode assembly 110 in the upward direction. Specifically, the positive electrode tab 134 may extend from the positive electrode 111 of the electrode assembly 110.

The positive electrode tab 134 may be connected to the cap plate 132, so that the upper cap 131 may function as a positive electrode terminal. An opening 151 is formed in the upper insulating member 150, and the positive electrode tab 134 may be connected to the cap plate 132 through the opening 151.

The center pin 140 generally includes a metal material to impart a certain strength and is formed as a cylindrical structure in which a plate is bent roundly. In addition to self-heating, the center pin 140 may fix and support the electrode assembly 110 and may function as a passage for releasing gas occurring due to an internal reaction during charging/discharging and operation.

An electrolyte injected into the battery case 120 may be a non-aqueous electrolyte containing a lithium salt, and the non-aqueous electrolyte containing a lithium salt includes a non-aqueous electrolyte and a lithium salt. Non-aqueous electrolytes include non-aqueous organic solvents, organic solid electrolytes, inorganic solid electrolytes, etc., but are not limited thereto.

In the cylindrical battery cell 100, a metal plate may be welded to electrically connect an electrode terminal of the cylindrical battery cell 100 exposed to the outside to an electrode terminal of another battery cell or a battery circuit.

A plurality of such cylindrical battery cells 100 may constitute a battery module or a battery pack.

In the thermal propagation (TP) test of the cylindrical battery cell 100, a method of igniting a cylindrical cell by heating the side of the cylindrical cell has been used in the related art, but the related art cylindrical cell ignition method causes a side rupture of the cylindrical cell when the cylindrical cell is ignited.

In an aspect of the present disclosure, a thermal propagation test method capable of igniting a cylindrical cell without causing a side rupture and a new heater used therefor are provided.

A heater for igniting a battery cell (cylindrical trigger cell) for a thermal propagation test and a thermal propagation test method using the same according to an aspect of the present disclosure are described in detail.

First, a heater 200 for igniting a battery cell (cylindrical trigger cell) for a thermal propagation test according to an aspect of the present disclosure is described with reference to the drawings.

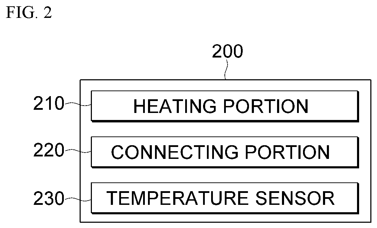

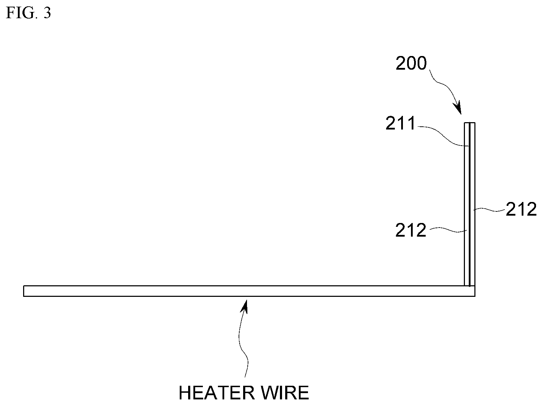

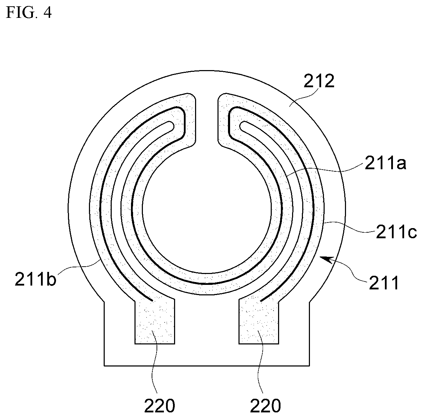

FIG. 2 is a diagram illustrating the heater 200 for igniting a battery cell for a thermal propagation test according to an aspect of the present disclosure, FIG. 3 is a diagram illustrating a side of the heater 200 illustrated in FIG. 2, and FIG. 4 is a diagram (plan view) illustrating a shape of the heater 200 illustrated in FIG. 2.

The heater 200 (hereinafter referred to as a 'heater') for igniting a battery cell (cylindrical trigger cell) for a thermal propagation test according to an aspect of the present disclosure may prevent a side rupture when the battery cell 100 (cylindrical trigger cell) for a thermal propagation test is ignited.

The battery cell 100 (or the cylindrical trigger cell, hereinafter collectively referred to as the battery cell 100) for a thermal propagation test may be a cylindrical battery cell and may include the electrode assembly 110 and the cylindrical battery case 120 that accommodates the electrode assembly 110.

Meanwhile, a safety vent which ruptures due to an increase of pressure inside the battery case 120 to discharge gas may be provided inside the battery case 120, for example, below the upper cap 131.

In the present disclosure, the heater 200 may be disposed below the battery cell 100 (cylindrical trigger cell) for a thermal propagation test and may heat the bottom of the battery cell 100 based on electricity supplied through a heater wire connected to the heater 200. Then, the battery cell 100 below which the heater 200 is placed may be ignited and the upper cap 131 of the battery cell 100 may be opened. For example, the heater 200 may have a thickness of about 1 mm and may be connected to a heater wire having a length of about 50 mm. In addition, the heater 200 may apply heat to the center of the bottom of the battery cell 100 (cylindrical trigger cell) based on electricity supplied through the heater wire.

In more detail, in the related art cylindrical cell ignition method, the related art heater is attached to the side of the cylindrical cell, so that a side rupture occurs near a heater attachment surface. Such side rupture occurs due to a local short occurring on the side of a cylindrical battery cell as the outermost separator near the heater attachment surface is damaged. Considering this point, the heater 100 according to an aspect of the present disclosure heats the center of the bottom surface of the cell in which there is a gap between the cylindrical can and the electrode, thereby damaging the inner separator, not the surface of the cell, and causing ignition, unlike the related art heater.

To this end, the heater 200 may include a heating portion 210, a connecting portion 220, and a temperature sensor 230, as illustrated in FIG. 2.

The heating portion 210 may be placed on a bottom surface of the battery cell 100 (cylindrical trigger cell) and may apply heat to the bottom surface of the battery cell 100.

Specifically, the heating portion 210 may include a heating member 211 and a heat-resistant sheet 212 as illustrated in FIGS. 3 and 4. The heating member 211 may generate heat upon receiving electricity through a heating wire. In the present aspect, the heating member 211 may include a first portion 211a and second portions 211b and 211c.

The first portion 211a may be formed in a circular shape with an open top, and the opposite ends located at the open top may be connected to the second portions 211b and 211c, respectively. That is, a left end among the opposite ends located at the open top of the first portion 211a may be connected to a left portion 211b among the second portions 211b and 211c, and a right end among the opposite ends located at the open top of the first portion 211a may be connected to a right portion 211c among the second portions 211b and 211c. A first temperature sensor 231 may be disposed at the inner center of the first portion 211a. That is, the first portion 211a of the heating member 211 may be disposed to surround the first temperature sensor 231.

The second portions 211b and 211c may be formed to surround the first portion 211a and may include the left portion 211b and the right portion 211c that are separated from each other. The left portion 211b and the right portion 211c may be formed in an approximately semicircular arc shape as illustrated.

The left portion 211b among the second portions 211b and 211c may be configured such that an upper end thereof is connected to the left end of the first portion 211a and surrounds the left portion of the first portion 211a. A lower end of the left portion 211b among the second portions 211b and 211c may be connected to the left connecting portion 220.

The right portion 211c among the second portions 211b and 211c may be configured such that an upper end thereof is connected to the right end of the first portion 211a and surrounds the right portion of the first portion 211a. A lower end of the right portion 211c among the second portions 211b and 211c may be connected to the right connecting portion 220.

The heating member 211 may be a heating metal, and the heating metal may be, for example, Inconel. A thickness of the heating member 211 may be, for example, about 0.1 mm, but the thickness may be changed.

In the present aspect, the heating portion 210 may be formed in a circular shape and may be coupled to the battery cell 100 to cover a portion of the bottom surface of the battery cell 100.

The heating portion 210 may be formed in a circular shape with a hollow space in the center and may be attached to the bottom surface of the battery cell 100. A first temperature sensor 231 may be mounted in the central empty space of the heating portion 210. For example, the heating portion 210 may have a diameter of about 16 mm, but the size may be changed.

The heat-resistant sheet 212 may be disposed on at least one surface of the heating member 211 and may be disposed between the heating member 211 and the bottom surface of the battery cell 100.

In the present aspect, the heat-resistant sheet 212 may be disposed on each of upper and lower surfaces of the heating member 211.

The heat-resistant sheet 212 may include a mica sheet, and a thickness thereof may be about 0.3 mm or 0.5 mm, but may be changed. The heat-resistant sheet 212 may cover the entire heating member 211 as illustrated in FIG. 4 and may extend toward the connecting portion 220 as well as the heating member 211 to cover the connecting portion 220.

In this manner, the heating portion 210 including the heating member 211 and the heat-resistant sheet 212 may be manufactured through a process of pressing the heating member 211 with two heat-resistant sheets 212.

In addition, the heating portion 210 may be attached to a lower surface of the battery cell 100 through a polyimide (PI) tape, and a thickness of the heating portion 210 may be about 1 mm, for example.

The connecting portion 220 may be connected to the heater wire at one end of the heating member 211. Specifically, the left connecting portion 220 among the two connecting portions 220 may extend in one direction from the lower end of the left portion 211b among the second portions 211b and 211c to be connected to the heater wire, and the right connecting portion 220 among the two connecting portions 220 may extend in one direction from the lower end of the right portion 211c among the second portions 211b and 211c to be connected to the heater wire.

Accordingly, the connecting portion 220 may connect the heating member 211 to the heater wire.

The two connecting portions 220 may extend in the same direction in parallel on the same plane as that of the heating member 211 and may be supplied with electricity from each heater wire.

The temperature sensor 230 may measure the temperature of the battery cell 100 and the heater 200 during the thermal propagation test.

To this end, the temperature sensor 230 may include a first temperature sensor 231 that measures the temperature of the heater 200 and a second temperature sensor 232 that measures the temperature of the battery cell 100.

The first temperature sensor 231 may be disposed in the central empty space of the heating portion 210 and may measure a temperature change of the heating portion 210 (heater 200) during the thermal propagation test.

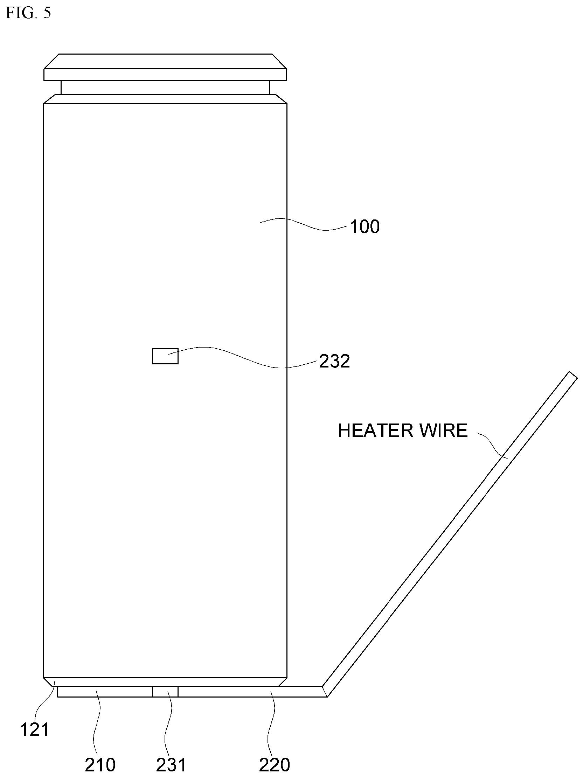

The second temperature sensor 232 may be disposed on the outer circumferential surface of the battery cell 100 (see FIG. 5) and may measure a temperature change of the battery cell 100 during the thermal propagation test.

Next, a method for testing thermal propagation of the battery cell 100 using the heater 200 for igniting a battery cell described above is described.

The method for testing thermal propagation of the battery cell 100 according to an aspect of the present disclosure may include an operation of disposing the heater 200 on the bottom surface of the battery cell 100 to heat the bottom surface of the battery cell 100; and an operation of igniting the battery cell 100 by heating the bottom surface of the battery cell 100 and may further include an operation of opening the top cap 131 of the battery cell 100 after the operation of igniting the battery cell.

FIG. 5 is a diagram illustrating an example in which the heater for igniting a battery cell is coupled to the battery cell 100 according to an aspect of the present disclosure.

Referring to FIG. 5, the heating portion 210 may be disposed on the bottom surface of the battery cell 100 to cover a portion of the bottom surface of the battery cell 100.

Also, in order to measure the temperature of the heater 200 during the thermal propagation test, the first temperature sensor 231 may be disposed in the central empty space of the heating portion 210 disposed on the bottom surface of the battery cell 100.

In addition, in order to measure the temperature of the battery cell 100 during the thermal propagation test, the second temperature sensor 232 may be mounted on the side of the battery cell 100.

Also, the connecting portion 220 may be connected to the heater wire.

Thereafter, when the thermal propagation test starts, the heating portion 210 heats the lower portion of the battery cell 100 upon receiving electricity from the heater wire through the connecting portion 220. Then, the battery cell 100 is continuously heated through the heating portion 210 to be ignited, and accordingly, the upper cap 131 of the battery cell 100 is opened.

In this manner, the heater 200 may cause normal ignition of the battery cell 100 that meets the international certification ISO6469-1 standard.

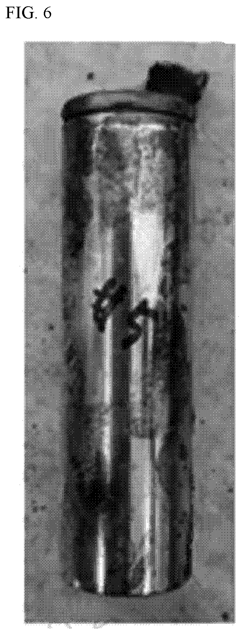

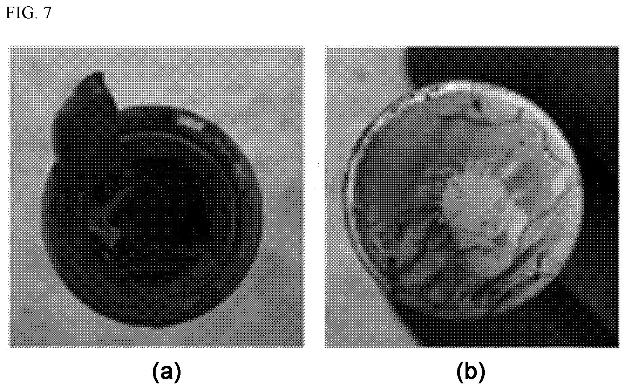

Meanwhile, photographs of the battery cell 100 after the test are illustrated in FIGS. 6 and 7.

FIG. 6 is a photograph of the battery cell 100 after the test, FIG. 7(a) is a photograph illustrating the upper portion of the battery cell 100 after the test, and FIG. 7(b) is a photograph illustrating the lower portion of the battery cell 100 after the test.

As illustrated in FIGS. 6 and 7, it can be seen that the side and lower surfaces of the battery cell 100 after the test were not damaged and that the upper cap 131 of the battery cell 100 was opened due to ignition.

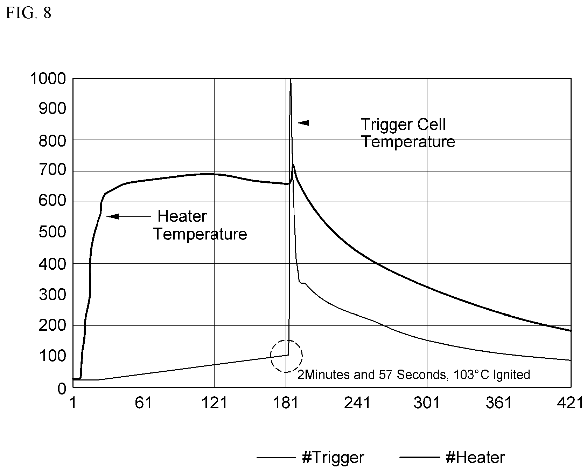

FIG. 8 is a graph illustrating the results of the thermal propagation test of the battery cell 100, in which the X-axis in FIG. 8 represents time (s) and the Y-axis represents temperature (°C).

Also, the orange line represents a temperature change of the heater 200 measured by the first temperature sensor 231, and the blue line represents a temperature change of the battery cell 100 measured by the second temperature sensor 232.

In this test, the heater 200 was heated from 0 °C to maintain at about 600 to 700 °C, and a current of 12 A was applied to the heater 200.

As illustrated in FIG. 8, an ignition temperature of the battery cell 100 was about 103 °C, and the time taken for ignition was about 2 minutes and 57 seconds.

The aspects of the present disclosure are intended to describe the technical idea, and the scope of the technical idea of the present disclosure is not limited by these aspects. The scope of the aspects of the present disclosure should be interpreted by the following claims, and all technical ideas within the equivalent scope should be interpreted as being included in the scope of the rights of the aspects of the present disclosure.

### [DESCRIPTION OF REFERENCE NUMERALS]

- 100 :: battery cell
- 200 :: heater
- 210 :: heating portion
- 220 :: connecting portion
- 230 :: temperature sensor

## Claims

1. A heater for igniting a battery cell for a thermal propagation test, the heater comprising:
a heating portion comprising a heater wire, the heating portion disposed on a bottom surface of the battery cell, wherein the heating portion is configured to heat the bottom surface of the battery cell; and
a connecting portion connected to the heater wire.

2. The heater of claim 1, wherein the heating portion has a circular shape.

3. The heater of claim 1, wherein the heating portion covers at least a portion of the bottom surface of the battery cell.

4. The heater of claim 1, wherein
the heating portion includes:
a heating member configured to heat the bottom surface of the battery cell; and
a heat-resistant sheet disposed on a surface of the heating member.

5. The heater of claim 4, wherein the heating member comprises a heating metal.

6. The heater of claim 5, wherein the heating metal is Inconel.

7. The heater of claim 4, wherein the heat-resistant sheet is disposed on an upper surface of the heating member and a lower surface of the heating member.

8. The heater of claim 4, wherein the heat-resistant sheet is a mica sheet.

9. The heater of claim 1, wherein the heating portion is attached to the battery cell by a polyimide (PI) tape.

10. The heater of claim 1, further comprising: a first temperature sensor configured to measure a temperature of the heater.

11. The heater of claim 10, further comprising: a second temperature sensor configured to measure a temperature of the battery cell.

12. The heater of claim 1, wherein the battery cell is a cylindrical battery cell.

13. A method for testing thermal propagation of a battery cell, the method comprising:
disposing a heater on a bottom surface of the battery cell;
heating the bottom surface of the battery cell; and
igniting the battery cell by heating the bottom surface of the battery cell.

14. The method of claim 13, wherein the igniting the battery cell opens a top cap of the battery cell.

15. The method of claim 13, wherein the battery cell is a cylindrical battery cell.
